# EUROPEAN PATENT SPECIFICATION

(11) **EP 1 489 962 B1**
(45) Date of publication and mention of the grant of the patent: **02.11.2006**
(21) Application number: 03721408.7
(22) Date of filing: 20.03.2003
(51) Int. Cl.: A61B 5/00, A61J 7/02

(54) **SYSTEM FOR MANAGING A PATIENT TREATMENT PROGRAM**
VORRICHTUNG ZUR VERWALTUNG VON EINEM PATIENTENBEHANDLUNGSPROGRAMM
SYSTEME DE GESTION D'UN PROGRAMME DE TRAITEMENT

(30) Priority: 28.03.2002 US 368250 P
(43) Date of publication of application: 29.12.2004
(73) Proprietor: ABBOTT LABORATORIES, Abbott Park, IL 60064-6008 (US)
(72) Inventor: MAYER, Steven, Lloyd, Salem, WI 53168 (US); URQUHART, John, Palo Alto, CA 94301 (US); METRY, Jean-Michel, CH-1950 Sion (CH); VRIJENS, Bernard, B-4690 (BE)
(74) Representative: Modiano, Micaela Nadia
(86) International application number: PCT/US2003/008580
(87) International publication number: WO 2003/082096

(56) References cited:
- US-A- 4 748 600
- US-A- 5 822 715

## Description

### Field of the Invention:

The invention pertains to a system and method for managing patient care associated with a prescribed drug regimen including predictive models used in combination with monitored compliance and testing.

### Background of the Invention:

Prescribed drugs can only be effective if properly taken. For many drugs there is often a finite usage range in which the drugs will produce the intended results. If not enough of a drug is taken, a drug may only be partially effective, may be non-effective, and/or may even promote undesirable effects. If too much of a drug is taken, undesirable side effects of the drug may manifest or become more pronounced.

One of the goals when prescribing medication in the treatment of a patient is to determine the proper amount of a drug, and the corresponding dosing interval, to produce the desired effect. However prescribing a proper amount of a drug and the related proper dosing interval is just part ofthe story. The patient then needs to take the medication as prescribed.

Many studies suggest that poor and partial adherence of patients to a prescribed drug regiment is prevalent, and many studies show that 50 percent or more of all patients prescribed drugs do not take them as prescribed. In a further study, of the number of doses prescribed, generally, one-third of the patients took greater than 95 percent of the prescribed doses, another third of the patients took between 70 and 95 percent of the prescribed doses, and the final third of the patients took fewer than 70 percent of the prescribed doses. Results indicative of poor or partial adherence are found even with life-saving treatment regimens, e.g., antiretroviral drug regimens prescribed in the treatment of an HIV infection. Poor and partial adherence for prescribed drug regimens also prevails to varying degrees for other types of chronic diseases or conditions, such as thyroid disease, hypertension, congestive heart failure, epilepsy, obesity and cancer.

Not only can patient compliance be a problem, but recognition of poor and partial compliance, in some instances, can go undetected by a care giver. In these instances, a poor response to a prescribed drug regimen can sometimes be falsely attributed to an inadequacy in the drug regimen. This in turn may prompt unnecessary changes to be made to the prescribed drug regimen, where sometimes the type or combination of drugs prescribed and/or the dosage levels may be altered. In some instances this may prompt a change in a drug regimen, which would otherwise have been effective, had proper compliance been maintained.

In an effort to detect poor compliance, and therefore minimize unnecessary changes, some care givers have instituted compliance monitoring as part of a prescribed drug regimen. One such approach includes tracking the number of doses taken during a prescribed period and comparing the number against the number of doses prescribed. However in using such an approach, an extra dose taken during one period can mask a missed dose in another period. Furthermore such an approach also fails to identify doses taken at the wrong time, where the doses may have been taken too late, whereby a longer period between doses occurs than was otherwise intended.

Because the drugs prescribed often have a relatively short half life, which relates to the time that the drug remains present in the patient's plasma and the corresponding concentration of the drug over time, large delays between doses and/or missed doses can create periods in which the drug concentration in the patient's plasma falls below levels needed for effective therapeutic action of the drug in question. In terms of the treatment of a viral infection, like HIV, ineffective concentrations, in addition to impacting the ability of the drug to suppress the virus, may create selection pressure, that encourages the emergence of a drug resistant strain. This undesirable situation occurs because drug levels, which promote only partial suppression, will generally have a greater impact on a strain of the virus that is non-resistant, as opposed to a strain of the virus that is more resistant to medication. Greater suppression of the non-resistant strain will allow a resistant strain to emerge and become dominant.

In an effort to more closely track patient compliance, monitoring systems have been developed, which not only track the number of doses taken over a predetermined period of time, but also keep track of the day and time each of the doses has been taken. For example, US-A-4 748 600 discloses an interactive drug dispenser which actively controls the pattern in which doses of one or more pharmaceutical preparations are administered to a patient. Another system is the MEMS® monitor produced by AARDEX Ltd. At least one version ofthe MEMS® monitor includes a cap closure adapted with sensors which detect the removal and the subsequent re-attachment of the cap from an enclosure containing the medication, and circuitry for recording the time and date when the cap is removed and re-attached. It is assumed that during each removal/re-attachment of the cap, a single dose of medication is dispensed from the enclosure and taken by the patient.

The monitored usage information can then be used in conjunction with predetermined characteristics of the prescribed medication, as well as the results of patient testing to make decisions concerning possible alterations in the patient's drug treatment program so as to provide safe and effective care.

However there is a cost associated with each activity, including a cost of the various tests to monitor the patient's condition, as well as a cost to performing the monitoring. Furthermore, the accepted characteristics of the prescribed medication, often relate to determined averages, some of which may or may not directly apply to a particular patient. Still further, given the number of variables involved, in monitoring, predicting, testing and interpreting the effects of the current prescribed dosing regimen, decisions concerning the need for adjustments in a patient's prescribed dosing regimen can be quite complex. This is further complicated by a desire to manage the patient's care, in a manner which is cost effective.

Consequently, it would be beneficial to develop a system and method for managing patient care associated with a prescribed drug regimen including predictive models used in combination with monitored compliance and testing. In at least some instances it would be beneficial to be able to individualize the predictive models and to be able to determine or confirm the accuracy of the models, as they relate to a particular patient, by correlating the predicted results with the measured response determined through testing, and to determine if and when testing should be performed for producing useful results.

Still further, it would similarly be beneficial to be able to determine a drug's effectiveness in producing a desired pharmacological effect over a broad range of patient adherence for determining the expected varying pharmacological impact of the drug as a function of change in adherence.

A method of individualizing the treatment of a patient associated with a prescribed drug regimen is provided. The method provides for the development of a pharmacokinetic model, which predicts the drug concentration over time in the patient in response to the drug dosage history of the patient, and the development of a pharmacodynamic model, which includes a predicted level of effectiveness for various levels of dosing and various degrees of deviation from the prescribed dosing regimen. A drug regimen is then prescribed for the patient, designed to achieve a desired pharmacological effect, based upon the pharmacokinetic model and the pharmacodynamic model.

The patient is then monitored to determine a degree of deviation from a prescribed dosing regimen. The pharmacological effect in the patient ofthe prescribed dosing regimen is then measured, and compared with the level of effectiveness that was predicted by the pharmacodynamic model, after taking into account the adherence of the patient to the prescribed dosing regimen.

If the measured effect deviates from the predicted level of effectiveness, the method then provides for additional tests to be performed to determine the actual drug concentration over time in the particular patient. The pharmacokinetic model is then adjusted based upon the actual determined drug concentration over time, and the prescribed drug regimen is adjusted for the patient to account for adjustments in the pharmacokinetic model.

Furthermore, an economic model is used to determine the most cost effective course in correcting for non-compliant patient behavior, if any. The economic model similarly enables the cost of the test to be compared against the likelihood of producing meaningful information, which can be used in verifying and adjusting the patient's present care, and in determining the order in which tests should be performed.

Furthermore, a method is provided for designing a clinical trial, which determines the effectiveness of a drug in producing a desired pharmacological effect over abroad range of patient adherence to a prescribed drug regimen, where the monitored adherence of one or more patients to the prescribed drug regimen, and the measured pharmacological effect in the one or more patients at various intervals, are related based upon the degree of deviation from the prescribed dosing regimen.

Furthermore, the methods and models are implemented as part of a system including a patient health management computer program comprising a communication unit for receiving access information indicative of patient compliance. The system further includes a processor for executing a plurality of prestored instructions, corresponding to creating and maintaining a pharmacokinetic model, a pharmacodynamic model and a decision analytic model. The system also includes an interface unit for communicating with a user the type and timing of tests recommended to be performed and for receiving the results of the tests.

### Summary of the invention:

According to the invention a system is provided for managing a patient treatment program comprising:
one or more enclosures each containing medication subscribed as part of a prescribed drug regimen for a respective one of the one or more patients, each enclosure being adapted for monitoring the access of the respective patient to the prescribed medication; and
a patient health management computer comprising
   a communication unit for receiving the access information from each of the one or more enclosures,
   a processor for executing a plurality of prestored instructions and data including
      instructions and data for creating and maintaining a pharmacokinetic model, which predicts the drug concentration over time in the patients, based at least in part upon the access information received from the corresponding enclosures,
      instructions and data for creating and maintaining a pharmacodynamic model, which receives the drug concentration over time predicted by the pharmacokinetic model, and predicts the level of effectiveness for various levels of dosing and various degrees of deviation from the prescribed dosing regimen, and
      instructions and data for creating and maintaining a decision analytic model, which receives the dosing history, the predicted drug concentration over time in the respective patients, and the predicted levels of effectiveness, for recommending when at least one of one or more tests should be performed on a patient to determine the actual condition of at least one aspect of the patient, and for deciding when the prescribed dosing regimen should be changed, and
   an interface unit for communicating to a user the recommendation when a test should be performed, and for receiving the results of the tests performed,
wherein the system is used for treating a patient with a viral infection, and the pharmacodynamic model includes a submodel for predicting the emergence of a drug resistant strain, or a submodel for predicting the response of CD4 cell counts to the prescribed drug regimen.

Numerous other advantages and features of the present invention will become readily apparent from the following detailed description of the invention and the embodiments thereof, from the claims and from the accompanying drawings.

### Brief Description of the Drawings:

Figure 1 is an example of a graph illustrating predicted drug concentration over time of the type that would be produced by a pharmacokinetic model;
Figure 2 is an example of a graph illustrating predicted viral suppression as a function of drug concentration of the type that would be produced by a pharmacodynamic model (confidential information communicated to Abbott by AARDEX Ltd);
Figure 3 is an example of a graph illustrating the likelihood of change in the condition of the patient, both positive and negative, based upon the degree of adherence to a prescribed drug regimen and the preceding condition of the patient (confidential information communicated to Abbott by AARDEX Ltd);
Figure 4 is a block diagram illustrating a model for use in managing a patient treatment program;
Figure 5 depicts an exemplary flow diagram of a method for individualizing the treatment of a patient associated with a prescribed drug regimen, for use with a model of the type illustrated in Figure 4;
Figure 6 depicts an exemplary flow diagram of a method for providing patient care, and for achieving and maintaining a level of wellness, for use with a model of the type illustrated in Figure 4;
Figure 7 depicts an exemplary flow diagram of the steps associated with determining the need for change in the drug regimen provided for in Figure 6;
Figure 8 depicts an exemplary flow diagram of a method for designing a clinical trial, which determines the effectiveness of a drug in producing a desired pharmacological effect over a broad range of patient adherence to a prescribed drug regimen, for use with a model of the type illustrated in Figure 4;
Figure 9 depicts an exemplary flow diagram of a method for managing an antiretroviral treatment program of a patient, for use with a model of the type illustrated in Figure 4; and
Figure 10 is a block diagram of one embodiment of a system for managing a patient treatment program on which at least portions of the model, illustrated in FIG. 4, and at least portions of the methods, illustrated in FIGS. 5-9, can be performed.

### Detailed Description of the Preferred Embodiments:

While the present invention is susceptible of embodiment in many different forms, there are shown in the drawings and will be described herein in detail specific embodiments thereof with the understanding that the present disclosure is to be considered as an exemplification of the principles of the invention and is not intended to limit the invention to the specific embodiments illustrated. The scope of the invention is defined in the claims.

The herein described system and method are well suited for managing a patient having a chronic disease or condition. One such condition for which the present system and method are particularly well suited is the management of a patient having an HIV infection. One such system and method of treating an HIV infection includes the use of one or more protease inhibitors for suppressing the virus, or in other word inhibiting the replication of the virus. While the present system and method are also applicable in managing the treatment of a patient having other types of chronic diseases or conditions, at times, the following description makes specific reference to an example including the use of an antiretroviral drug regimen in the treatment of an HIV infection, which is presently viewed as corresponding to and illustrative of the preferred embodiment.

A chronic disease is broadly defined as an illness that is prolonged, does not resolve spontaneously, is rarely cured completely, and requires persistent administration of one or more prescription drugs to main tain the patient in a preferred medical status. Specific examples of other types of chronic diseases and conditions, in addition to HIV, for which the present invention has been identified as being particularly applicable include thyroid disease, hypertension, congestive heart failure, epilepsy, obesity and cancer. For example, in the treatment ofthyroid disease, patient compliance with the administration of thyroxine or triiodothyronine may be managed; in the treatment of hypertension, patient compliance with the administration of diuretics, and antihypertensive agents such as trandolapril, captopril, enalapril, betaxolol, propranolol, atenolol, metoprolol, nifedipine, verapamil, diltiazem, hydrochlorothiazide, and the like may be managed; in the treatment of congestive heart failure, patient compliance with the administration of furosemide, digoxin, potassium salts, and others may be managed; in the treatment of obesity, patient compliance with the administration of sibutramine maybe managed; and in the treatment of cancer, patient compliance with the administration oftamoxifen and other agents designed for administration by patients may be managed.

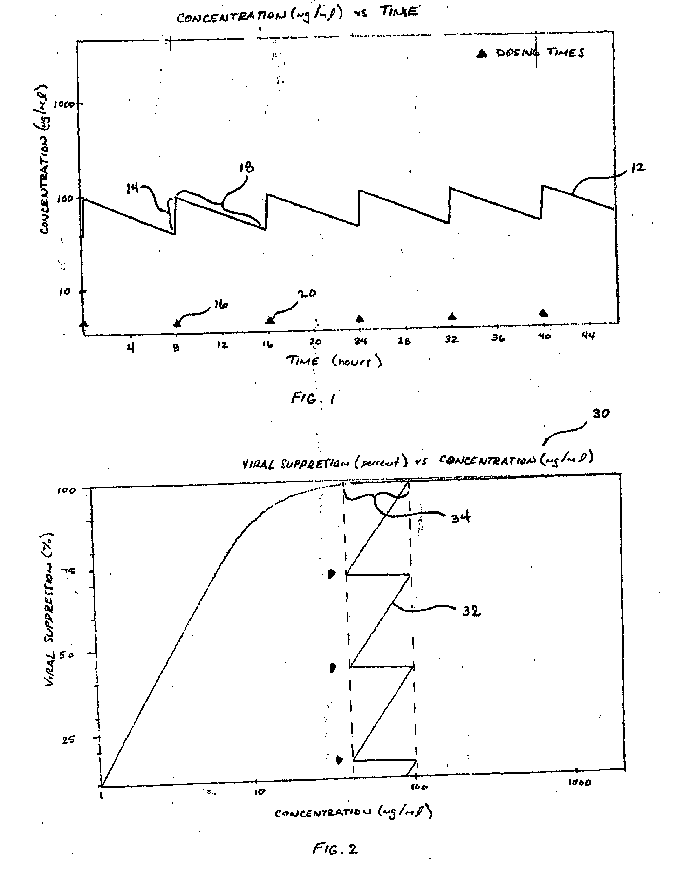

Figure 1 illustrates a graph 10 depicting predicted drug concentration as a function of time of the type that would be produced by a pharmacokinetic model. Generally, the model depicts a prescribed medication being taken at periodic intervals during which the concentration levels 12 of the drug in the plasma of the patient changes over time. The drug levels are typically initially boosted 14 shortly after a dose 16 is taken by the patient, and then gradually declines 18 up until the time proximate to the patient taking the next dose 20.

The specific drug levels are affected by the rate at which the drug is absorbed, distributed, metabolized, and excreted by a patient. In practice the actual rate at which the drug is absorbed, distributed, metabolized, and excreted, can vary between patients. At least initially, a model will generally be reflective of the expected average across all patients. Consequently, if one or more of the specific parameters for a particular patient vary sufficiently away from the average, either separately or in combination, the model based upon the average may not be reflective of the actual drug behavior in the particular patient. Correspondingly, the model may need to be adjusted for a particular patient.

As concentration of the drug varies in the plasma (and sometimes in other fluids and tissues), often times the drug's effectiveness similarly varies. A pharmacodynamic model is intended to express the relationship between drug concentrations in the patient and a resulting pharmacological effect. One such pharmacological effect related to drug concentration is illustrated in Figure 2.

Figure 2 illustrates a graph 30 depicting viral suppression or inhibition ofreplication of a virus as a percentage for one type of protease inhibitor as against at least one strain of the virus. Generally, as the concentration of the drug increases, the drugs effectiveness in-inhibiting replication similarly increases. Overlayed upon the graph 30 is the data corresponding to drug concentration levels 32, illustrated in Figure 1. Over the anticipated range 34 of drug concentration levels, the drug effectiveness varies between approximately 97-99 percent. However one notices that if concentrations were allowed to fall further, that the decrease in effectiveness begins to accelerate.

Different strains of the virus can experience different levels of impact from varying levels of drug concentration for a particular drug. In connection with treating HIV infections, one area of concern is the emergence of drug resistant strains, that can result from suboptimal levels of treatment. At some drug concentrations, a drug may continue to be very effective against the non-drug resistant version of the strain, but begin to experience a substantial drop-off in effectiveness against resistant strains of the virus. In these circumstances the likelihood of a drug resistant strain emerging becomes more likely.

Ideally, the prescribed drug regimen is designed to provide drug concentrations that are substantially effective against both resistant and non-resistant strains. However the difficulty arises when individual or multiple doses of the prescribed drug regimen are missed or delayed thereby allowing the drug concentrations to dip further than intended. Under these circumstances the emergence of a drug resistant strain may become increasingly possible.

The emergence of a drug resistant strain is one of many pharmacological effects that can be modeled as part of a pharmacodynamic model. Still further it is possible using a pharmacodynamic model to track multiple pharmacological effects. Examples of additional pharmacological effects, which are incorporated as part of at least one of the preferred embodiments of the present invention include the effects of drug concentration levels on viral load, and the effects on CD4 cell counts as a result of maintaining a certain level of drug concentration, as part of the drug regimen.

As briefly noted above, missed or delayed doses can have a profound effect on the effectiveness of the drug in promoting the desired pharmacological effect. Despite Figure 1 illustrating drug levels resulting from good patient compliance, perfect patient compliance rarely, if ever, occurs.

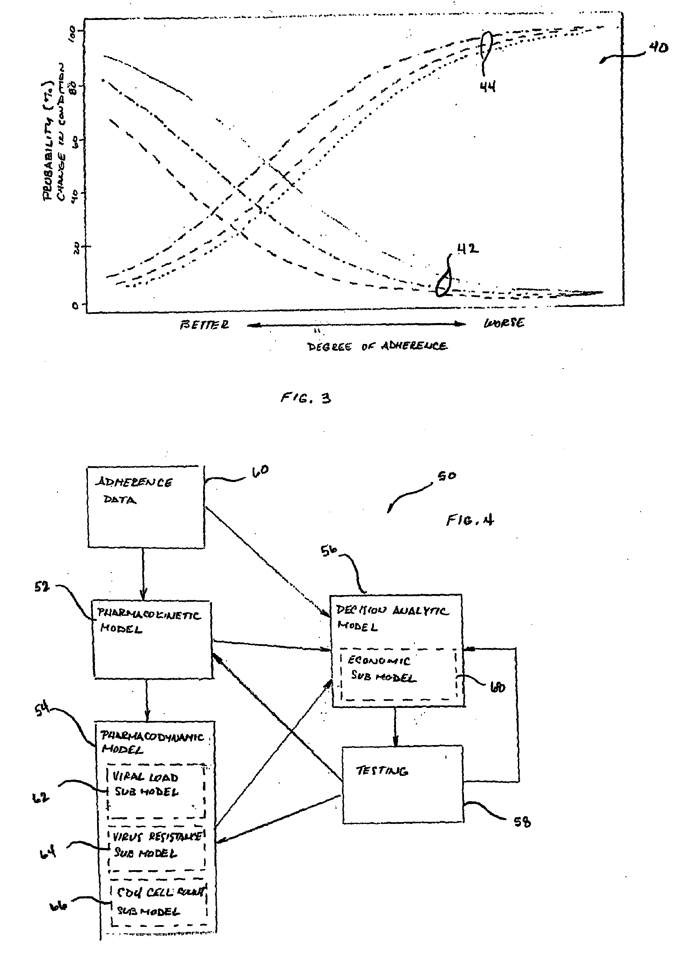

Figure 3 illustrates a graph 40 where expected changes in a patient condition are tracked as a function of patient compliance or adherence to the prescribed drug regimen. Multiple overlaid graphs represent the likelihood of improvement and likelihood that the condition will become worse, based upon different starting conditions. As is generally the case in HIV infections, the worse the condition of the patient is when the treatment starts, the greater the opportunities to induce improvements in the patient's condition. Generally the converse is similarly true.

In graph 40 a first set of lines 42 represents the predicted likelihood that the patient's condition will improve. A second set of lines 44 represents the predicted likelihood that the patient's condition will become worse. In the case of the first set of lines 42, the top line represents a starting condition for the patient in which the viral load count is initially higher than the other two lines from the group 42. The converse is generally true with respect to the second set of lines 44, i.e. that you have a greater chance of becoming worse, if your initial condition is better.

Furthermore, the greater the deviation from optimal dosing levels the greater the likelihood of negatively impacting the chances for improvement.

Graph 40 can be used to anticipate different responses to varying levels of treatment and varying levels of compliance. An estimate as to the impact to the patient given anticipated or proposed changes in patient compliance can be quantified, which allows for cost benefit analysis to be more easily applied.

In some instances, patient compliance can be increased by intervening with the patient when non-compliance is detected. For example, explaining the consequences as to overall health of non-compliant behavior is sometimes sufficient for having an effect. Depending upon where the patient is along the curve will determine how significant of an impact a change in compliance is likely to be. At some point, the benefits may be significant enough in terms of promoting wellness that it is warranted to incur a higher degree of intervening costs. In these instances it may be worthwhile to monitor in real time the patient's dosing history, and when a delayed or missed dose is detected, page or call the patient. In other instances it may be more cost effective to adjust the patient's subsequent dosing to accommodate one or more missed doses.

By combining the multiple models and monitoring a patient's adherence to a prescribed drug regimen, the ability to develop an effective treatment program is greatly improved. In addition to being able to better predict the likely results of the treatment in the patient, the combined models can be used to predict when actual testing of the patient is likely to yield data that can be used to confirm the accuracy of the models and the corresponding effectiveness of the prescribed treatment, and/or identify other more serious issues.

Figure 4 illustrates a model 50 for use in managing the treatment of a patient including a combination of a pharmacokinetic model 52, a pharmacodynamic model 54, and a decision analytic model 56, as well as provisions 58 for requesting that tests be performed and for receiving the test results, and provisions for receiving adherence data 60.

Generally, the adherence data 60 are received and provided to the pharmacokinetic model 52. The pharmacokinetic model 52 produces predicted drug concentrations, and supplies the same to the pharmacodynamic model 54. The pharmacodynamic model 54, produces a prediction as to one or more pharmacological effects including predictions as to viral load as part of a viral load submodel 62, the emergence of viral resistance as part of a virus resistance submodel 64, and a CD4 cell count as part of a CD4 cell count submodel 66. CD4 cell counts can be very useful in determining the likelihood of opportunistic infections, and in making the decision to prescribe additional medication to ward off the same.

All ofthe data are made available to the decision analytic model 56, which in turn can determine when to recommend that certain testing be performed, and can even base the decision upon rational economics using an economic submodel 68.

The results of the tests can then be used to update the model and fine tune the models to the individual patient, as well as to make determinations concerning additional recommended tests.

In at least one embodiment the combined model 50 is implemented at least in part using a computer. An example of one such system is described below in connection with Figure 10.

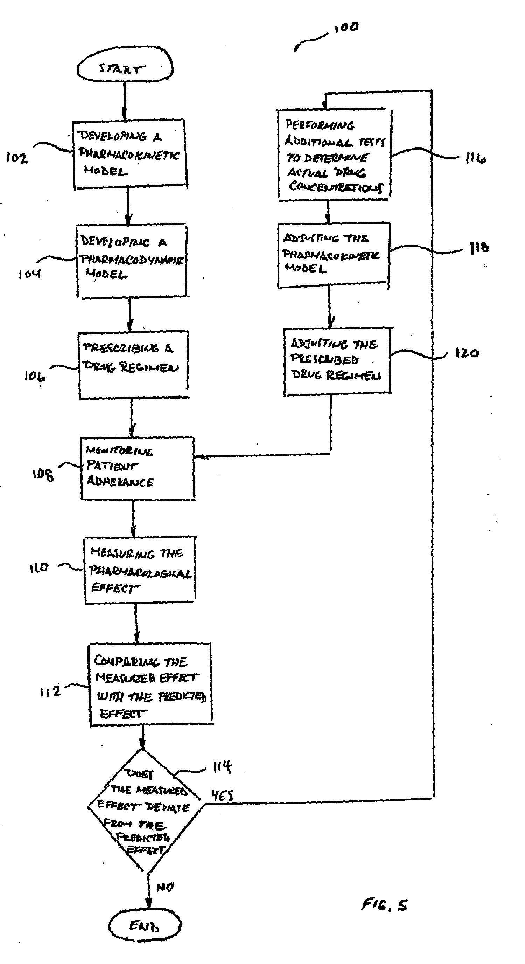

Figure 5 depicts an exemplary flow diagram of a method 100 for individualizing the treatment of a patient associated with a prescribed drug regimen, for use with a model 50 of the type illustrated in Figure 4. The method 100 provides for initially developing 102 both a pharmacokinetic model, which predicts the drug concentration over time in the patient in response to the drug dosage history of the patient, and developing 104 a pharmacodynamic model, which includes a predicted level of effectiveness for various levels of dosing and various degrees of deviation from a prescribed drug regimen. Generally both a pharmacokinetic model and a pharmacodynamic model can be developed as part of clinical trial. However previous clinical trials have generally not separately determined effectiveness, based upon patient adherence.

A drug regimen is then prescribed 106. The adherence to the prescribed drug regimen is then monitored 108. Testing is then performed to measure 110 the pharmacological effect of the drug dosing regimen. The measured effect is then compared 112 with the effect predicted by the pharmacodynamic model after taking into account the adherence data of the patient. Taking into account the adherence data can be important, because as noted above, the actual adherence can have a profound effect, and may be able to explain poor results.

If the measured effect deviates from the expected result 114, even after taking into account the adherence of the patient, then the method provides for performing 116 additional tests for determining actual drug concentrations. A common test for determining the actual drug concentrations is known as therapeutic drug monitoring. Such a test can determine if this particular patient is not well represented by the general pharmacokinetic model directed to the average patient.

If the test results suggest that the pharmacokinetic model fails to provide an adequate prediction for this particular patient, a determination is then made as to what changes need to be made to the pharmacokinetic model, and the adjustments are made 118. The prescribed drug regimen is then adjusted 120 accordingly. In this way, a method 100 of individualizing the treatment of a patient can be accomplished.

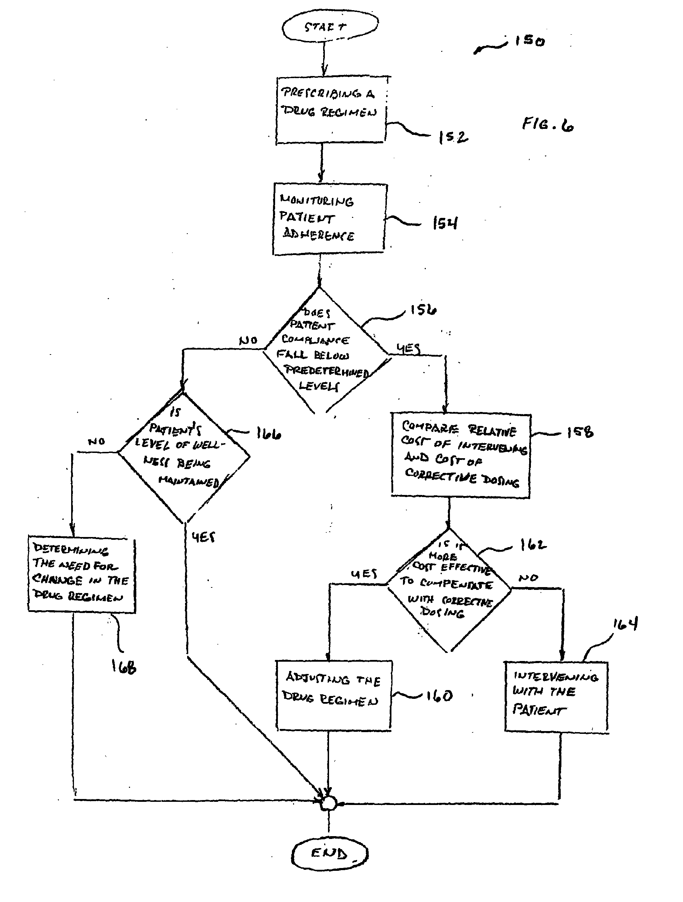

Figure 6 depicts an exemplary flow diagram of a method 150 for providing patient care, and for achieving and maintaining a level of wellness, for use with a model of the type illustrated in Figure 4. Initially, a drug regimen is prescribed 152, that is directed to achieving and maintaining a predetermined level of wellness. The adherence of the patient to the drug regimen is then monitored 154. A determination 156 is then made as to whether compliance levels are being maintained at satisfactory levels. If the level of compliance falls below the satisfactory levels, a determination is made of the anticipated cost to compensate for non-compliant behavior, and the cost for corrective drug dosing is compared against the cost of intervening with the patient 158.

The method then provides for adjusting the drug regimen 160, if it is determined to be more cost effective 162. Alternatively, if the available intervention alternatives are more cost effective, the method then provides for intervening with the patient 164.

As noted previously, intervening activity can include paging or calling the patient when non-compliance is detected. It can also include patient education concerning the significance and effect of non-compliance. Initially lower cost interventions can be tried and the adherence monitored to determine if the intervention was successful. Later more expensive interventions can be attempted, if necessary, and if it is estimated that they will be more cost effective than corrective dosing.

If the patient's adherence is good, but the predetermined level of wellness fails to be maintained 166, then the model determines 168 whether there is a need for a change in the prescribed drug regimen.

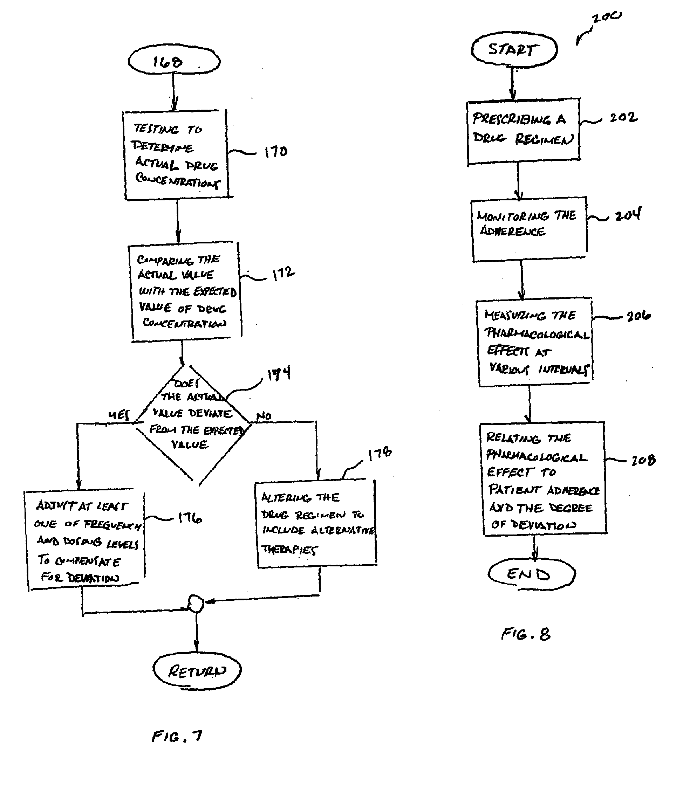

Figure 7 depicts an exemplary flow diagram of the steps associated with determining the need for change in the drug regimen 168 provided for in Figure 6. Initially, testing is performed 170 to determine the actual drug concentrations in the patient. As noted previously, sometimes the pharmacokinetic model needs to be adjusted for a particular patient. The actual level of drug concentration over time is then compared 172 against the expected values predicted by the pharmacokinetic model. If the actual drug concentration levels deviate from the expected value 174, the method then adjusts 176 at least one of the dosing frequency and dosing levels to compensate for the deviation.

If actual drug concentration levels are in line with expected drug concentration levels, then the method provides for altering 178 the drug regimen to include alternative therapies. In the case of treating an HIV infection, another drug could be prescribed for which the patient's form of the virus has not developed a resistance.

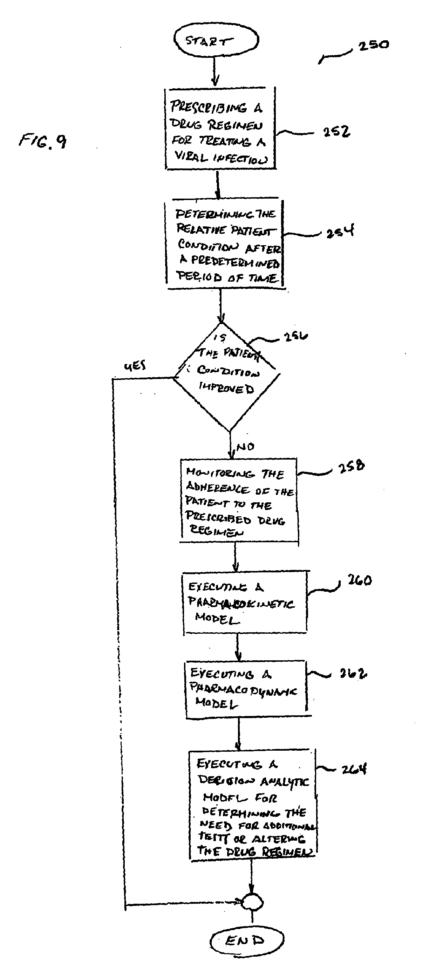

Figure 8 depicts an exemplary flow diagram of a method 200 for designing a clinical trial, which determines the effectiveness of a drug in producing a desired pharmacological effect over a broad range of patient adherence to a prescribed drug regimen, for use with a model of the type illustrated in Figure 4. Initially a drug regimen is prescribed 202 to one or more patients. The adherence to the prescribed drug regimen for each of the one or more patients is then monitored 204. The pharmacological effect for each of the one or more patients is then measured 206 at various intervals. The measured pharmacological effect is then related 208 to the patient adherence data for determining the pharmacological effect over a broad range of patient adherence to a prescribed drug regimen.

The method 200 benefits from the inherent variability in patient adherence, and in turn uses the resulting test data as useful information from which future results can be predicted, based upon broader ranges of adherence. Where the monitored adherence is at a level for which insufficient predictive data exists, the method could prompt the patient for additional testing.

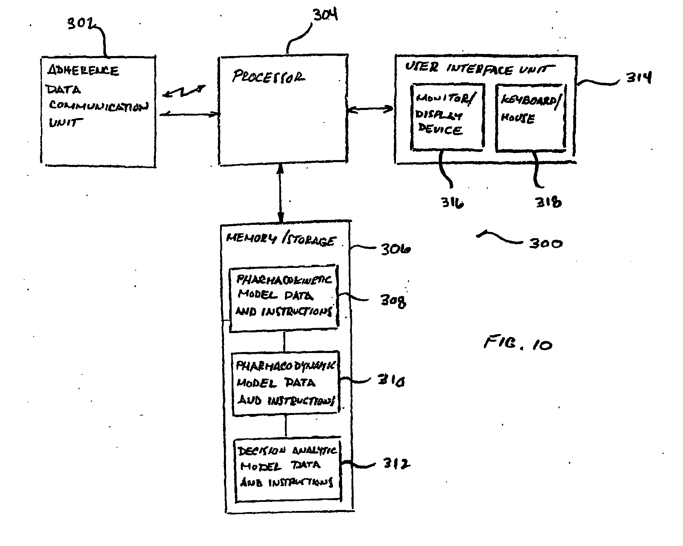

Figure 9 depicts an exemplary flow diagram of a method 250 for managing an antiretroviral treatment program of a patient, for use with a model of the type illustrated in Figure 4. Initially, a drug regimen is prescribed 252 for treating a viral infection. The condition of the patient is then determined after a predetermined period of time 254. If the patient's condition has improved 256, then no changes are made to the regimen.

If the patient's condition has not improved 256, then the method provides for the monitoring 258 of the adherence of the patient to the prescribed drug regimen. A pharmacokinetic model is then executed 260, in conjunction with a pharmacodynamic model 262. The method then further executes 264 a decision analytic model for determining the need for additional tests or for determining the need to alter the drug regimen.

While previously it has been noted that it may be desirable to update a pharmacokinetic model, so as to more closely correspond to a particular patient, it is also possible that the pharmacodynamic model should be updated to account for characteristics unique to the patient. Correspondingly the results of the viral resistance testing or other related testing might suggest, or make desirable, that the pharmacodynamic model be updated.

Figure 10 is a block diagram of one embodiment of a system 300 for managing a patient treatment program on which at least portions of the model, illustrated in FIG. 4, and at least portions of the methods, illustrated in FIGS. 5-9, can be performed. The system 300 includes a patient health management computer including an adherence data communication unit 302. The communication unit 302 can take the form of several well known communication interfaces for a computer of the type including a modem, a radio transceiver, a serial or parallel interface, a SCSI adapter, a USB adapter, and network interface card. In at least one embodiment the communication unit 302 includes an interface cradle for receiving one or more of the enclosures serving as a compliance monitoring device. Alternatively the communication unit 302 could receive the data wirelessly. In at least one embodiment, the noted enclosures could take the form of the MEMS® monitoring device discussed in the background of the art section.

The system 300 further includes a processor 304 for executing a plurality of prestored instructions. The instructions are generally stored in some form in memory, such as ROM or RAM, or as part of some auxiliary storage device, such as an optical disk, a hard disk, or a floppy disk. The memory/storage 306 in which the operating instructions and corresponding data are stored can be integral to the processor, or part of a separate connected unit.

The stored instructions and data include instructions 308 for creating and maintaining a pharmacokinetic model, instructions 310 for creating and maintaining a pharmacodynamic model, and instructions 312 for creating and maintaining a decision analytic model.

The system 300 still further includes a user interface unit 314 for communicating to a user any recommendation as to when an action should be performed related to the management of patient care. Such actions could include prompting for a test to be performed, and indications that the patient needs to be contacted concerning a reminder to take his/her medication. The communication could be displayed on a monitor or display device 316. The communication could alternatively be communicated audibly through a speaker.

The user interface unit 314 additionally enables the user to supply data to the computer. Traditionally such communication has been performed through devices such as a keyboard, a mouse or other pointing device 318. Other forms of user interface devices include touch screens, or microphones. One skilled in the art will readily recognize other forms of communication through other types of user interface devices are additionally available between a user and a computer, without departing from the scope of the present invention.

In another embodiment the method and system of the present invention may be used for the management of thyroid diseases. For example, a drug such as thyroxine may be packaged in a dispenser such as a blister pack or circular dial pack with a child resistant housing or closure and a MEMS® monitor. Alternatively, a stackable magazine like dispenser with pills in a size and shape for that dispenser may be used.

Monitoring and patient prompts may be utilized to individualize dosing and therapy when used in conjunction with measurement of a patient's thyroid hormone level.

Use of such a system will encourage improved patient compliance or permit the modification of dose in view of the patient's compliance history.

From the foregoing, it will be observed that numerous variations and modifications may be effected.

The scope of the invention is defined in the claims.

## Claims

1. A system (300) for managing a patient treatment program comprising:
one or more enclosures each containing medication subscribed as part of a prescribed drug regimen for a respective one of the one or more patients, each enclosure being adapted for monitoring the access of the respective patient to the prescribed medication; and
a patient health management computer comprising
a communication unit (302) for receiving the access information from each of the one or more enclosures,
a processor (304) for executing a plurality of prestored instructions and data including
instructions (308) and data for creating and maintaining a pharmacokinetic model, which predicts the drug concentration over time in the patients, based at least in part upon the access information received from the corresponding enclosures,
instructions (310) and data for creating and maintaining a pharmacodynamic model, which receives the drug concentration over time predicted by the pharmacokinetic model, and predicts the level of effectiveness for various levels of dosing and various degrees of deviation from the prescribed dosing regimen, and
instructions (312) and data for creating and maintaining a decision analytic model, which receives the dosing history, the predicted drug concentration over time in the respective patients, and the predicted levels of effectiveness, for recommending when at least one of one or more tests should be performed on a patient to determine the actual condition of at least one aspect of the patient, and for deciding when the prescribed dosing regimen should be changed, and
an interface unit (314) for communicating to a user the recommendation when a test should be performed, and for receiving the results of the tests performed,
wherein the system is adapted for treating a patient with a viral infection and the pharmacodynamic model includes a submodel for predicting the emergence of a drug resistant strain, or a submodel for predicting the response of CD4 cell counts to the prescribed drug regimen.

2. The system (300) of claim 1, wherein the enclosure includes a reservoir encapsulating a space capable of holding one or more doses of a medication, said reservoir having an opening through which access to the one or more doses is possible, and a cap, which selectively covers said opening, and which is adapted to detect its position relative to the reservoir between covering said opening and not covering said opening.

3. The system (300) of claim 2, wherein the cap includes a calendar and clock for detecting when the patient accesses the enclosure.

4. The system (300) of claim 2, wherein the cap includes a memory for storing the access data for each instance that the patient accesses the enclosure.

5. The system (300) of claim 2, wherein the cap includes a transmitter and a receiver for wirelessly communicating the access data with the communication unit of the computer data server.

6. The system (300) of claim 1, wherein the prescribed drug regimen includes the use of an antiretroviral agent.

7. The system (300) of claim 1, wherein the one or more tests include at least one of therapeutic drug monitoring for measuring the actual drug concentrations over time in the patient, a viral load test for measuring the actual amount of virus present in the patient, a drug resistance test for detecting the emergence of a drug resistant strain of the virus and measuring the relative amount of the drug resistant strain, and a CD4 count for measuring the absolute and relative amounts of CD4 cells in the plasma of the patient.

## Patentansprüche

1. Ein System (300) zur Verwaltung eines Patientenbehandlungsprogramms, das Folgendes umfasst:
einen oder mehrere Behälter, von denen jeder ein Medikament enthält, das als Teil eines vorgeschriebenen Arzneimittelregimes für einen entsprechenden der einen oder mehreren Patienten verschrieben wird, wobei jeder Behälter ausgebildet ist, um den Zugriff des jeweiligen Patienten auf das verschriebene Medikament zu überwachen; und
einen Patienten-Gesundheitsüberwachungs-Computer, der Folgendes umfasst:
eine Kommunikationseinheit (302), um die Zugriffsinformationen von jedem der einen oder mehreren Behältern zu empfangen,
einen Prozessor (304) zur Ausführung einer Vielzahl zuvor gespeicherter Anweisungen und Daten, der Folgendes einschließt:
Anweisungen (308) und Daten zur Erstellung und Verwaltung eines pharmakokinetischen Modells, das die Arzneimittelkonzentration im Verlauf der Zeit bei den Patienten überwacht, zumindest teilweise auf den Zugriffsinformationen basierend, die von den entsprechenden Behältern empfangen werden,
Anweisungen (310) und Daten zur Erstellung und Verwaltung eines pharmakodynamischen Modells, das die Arzneimittelkonzentration im Verlauf der Zeit empfängt, die vom pharmakokinetischen Modell prognostiziert wird, und das den Grad der Wirksamkeit für verschiedene Dosierniveaus und verschiedene Grade der Abweichung vom vorgeschriebenen Dosierregime prognostiziert, und
Anweisungen (312) und Daten zur Erstellung und Verwaltung eines analytischen Entscheidungs-Modells, das die Dosierungsgeschichte, die prognostizierte Arzneimittelkonzentration im Verlauf der Zeit bei den jeweiligen Patienten und die prognostizierten Wirksamkeitsgrade empfängt, um zu empfehlen, wann mindestens einer der einen oder mehreren Tests bei einem Patienten durchgeführt werden sollte, um den eigentlichen Zustand mindestens eines Aspekts des Patienten zu bestimmen und um zu entscheiden, wann das vorgeschriebene Dosierregime geändert werden sollte, und
eine Schnittstelleneinheit (314), um einem Benutzer die Empfehlung mitzuteilen, wann ein Test durchgeführt werden sollte, und um die Ergebnisse der durchgeführten Tests zu empfangen,
worin das System ausgebildet ist, um einen Patienten mit einer viralen Infektion zu behandeln, und das pharmakodynamische Modell ein Teilmodell zur Prognostizierung des Auftretens eines arzneimittelresistenten Stammes oder ein Teilmodell zur Prognostizierung der Reaktion von CD4-Zellzählungen auf das vorgeschriebene Arzneimittelregime einschließt.

2. Das System (300) von Anspruch 1, worin der Behälter ein Reservoir einschließt, das einen Raum umhüllt, welcher eine oder mehrere Dosen eines Medikaments enthalten kann, wobei das Reservoir eine Öffnung hat, durch welche der Zugriff auf die eine oder mehreren Dosen möglich ist, und eine Kappe, welche die Öffnung selektiv bedeckt und ausgebildet ist, um ihre Position relativ zum Reservoir zwischen der Abdeckung der Öffnung und der fehlenden Abdeckung der Öffnung zu erkennen.

3. Das System (300) von Anspruch 2, worin die Kappe einen Kalender und eine Uhr zum Nachweis, wann der Patient auf das Reservoir zugreift, einschließt.

4. Das System (300) von Anspruch 2, worin die Kappe einen Speicher zum Speichern der Zugriffsdaten für jedes Mal, wenn der Patient auf das Reservoir zugreift, einschließt.

5. Das System (300) von Anspruch 2, worin die Kappe einen Sender und einen Empfänger einschließt, um die Zugriffsdaten drahtlos an die Kommunikationseinheit des Computer-Datenservers zu übertragen.

6. Das System (300) von Anspruch 1, worin das vorgeschriebene Arzneimittelregime die Verwendung eines antiretroviralen Mittels einschließt.

7. Das System (300) von Anspruch 1, worin der eine oder die mehreren Tests mindestens eines der Folgenden einschließen: therapeutische Arzneimittel-Überwachung zur Messung der tatsächlichen Arzneimittelkonzentrationen im Verlauf der Zeit bei einem Patienten, ein Virenlast-Test zur Messung der tatsächlichen Menge der Viren, die beim Patienten vorhanden sind, einen Arzneimittelresistenz-Test zum Nachweis des Auftretens eines arzneimittelresistenten Stamms des Virus und zur Messung der relativen Menge des arzneimittelresistenten Stamms, und eine CD4-Zählung zur Messung der absoluten und der relativen Menge von CD4-Zellen im Plasma des Patienten.

## Revendications

1. Système (300) destiné à gérer un programme de traitement de patient, comprenant :
une ou plusieurs enveloppes contenant chacune un médicament prescrit faisant partie d'un régime médicamenteux prescrit pour un patient respectif des un ou plusieurs patients, chaque enveloppe étant conçue pour contrôler l'accès du patient respectif au médicament prescrit ; et
un ordinateur de gestion de la santé de patients, comprenant :
une unité de communication (302) pour recevoir les informations d'accès de chacune des une ou plusieurs enveloppes,
un processeur (304) pour exécuter une pluralité d'instructions et de données mémorisées au préalable comprenant
des instructions (308) et des données pour créer et conserver un modèle pharmacocinétique, qui prédit la concentration de médicament avec le temps chez les patients, en se basant au moins en partie sur les informations d'accès reçues des enveloppes correspondantes,
des instructions (310) et données pour créer et conserver un modèle pharmacodynamique, qui reçoit la concentration de médicament avec le temps prédite par le modèle pharmacocinétique, et prédit le niveau d'efficacité de divers niveaux de dosage et divers degrés d'écart par rapport au régime de dosage prescrit, et
des instructions (312) et données pour créer et conserver un modèle analytique de décision, qui reçoit l'historique de dosage, la concentration de médicament prescrite avec le temps chez les patients respectifs, et les niveaux prédits d'efficacité, pour recommander un moment où au moins un ou plusieurs essais doivent être effectués sur le patient pour déterminer l'état réel d'au moins un aspect du patient, et pour décider le moment où le régime de dosage prescrit doit être modifié, et
une unité d'interface (314) pour communiquer à un utilisateur la recommandation indiquant le moment où un essai doit être effectué, et pour recevoir les résultats des essais effectués,
dans lequel le système est adapté pour traiter un patient ayant une infection virale, et le modèle pharmacodynamique comprend un sous-modèle pour prédire l'émergence d'une souche pharmacorésistante, ou un sous-modèle pour prédire la réaction de numérations de cellules CD4 au régime médicamenteux prescrit.

2. Système (300) selon la revendication 1, dans lequel l'enveloppe comprend un réservoir encapsulant un espace capable de contenir une ou plusieurs doses d'un médicament, ledit réservoir ayant une ouverture à travers laquelle il est possible d'accéder à la dose ou aux doses, et un obturateur, qui recouvre sélectivement ladite ouverture, et qui est adapté pour détecter sa position par rapport au réservoir entre la couverture dudit réservoir et la non-couverture dudit réservoir.

3. Système (300) selon la revendication 2, dans lequel l'obturateur comprend un calendrier et une horloge pour détecter le moment où le patient accède à l'enveloppe.

4. Système (300) selon la revendication 2; dans lequel l'obturateur comprend une mémoire pour stocker les données d'accès chaque fois que le patient accède à l'enveloppe.

5. Système (300) selon la revendication 2, dans lequel l'obturateur comprend un émetteur et un récepteur pour une communication sans fil des données d'accès à l'unité de communication du serveur de données informatiques.

6. Système (300) selon la revendication 1, dans lequel le régime médicamenteux prescrit comprend l'utilisation d'un agent antirétroviral.

7. Système (300) selon la revendication 1, dans lequel le test ou les tests incluent au moins un d'un contrôle du médicament thérapeutique pour mesurer les concentrations réelles du médicament avec le temps chez le patient, d'un essai de charge virale pour mesurer la quantité réelle de virus présent chez le patient, un essai de résistance au médicament pour détecter l'émergence d'une souche pharmacorésistante et mesurer la quantité relative de la souche pharmacorésistante, et une numération de CD4 pour mesurer les quantités absolue et relative de cellules CD4 dans le plasma du patient.
